# EUROPEAN PATENT APPLICATION

(11) **EP 3 513 871 A1**
(43) Date of publication of application: **24.07.2019**
(21) Application number: 18152110.5
(22) Date of filing: 17.01.2018
(51) Int. Cl.: B01J 4/00, C07C 273/04, B01J 19/24, B01J 10/00

(54) **CARBAMATE DECOMPOSER COMPRISING FLOW DISTRIBUTOR**

(71) Applicant: Yara International ASA, 0277 Oslo (NO)
(72) Inventor: PORRO, Lino Giovanni, 1040 Etterbeek (BE); SERRAIOCCO, Luigi, 3929 Porsgrunn (NO)
(74) Representative: De Clercq & Partners

(57) **Abstract**

The present application relates to a carbamate decomposer, comprising an inlet for a urea-water-carbamate mixture, an outlet for a two-phase flow comprising liquid urea, water, un-decomposed carbamate, gaseous ammonia, and gaseous carbon dioxide, a decomposition section, and a fluid distribution section, wherein the fluid distribution section comprises a conical flow distributor, the conical flow distributor comprising a plurality of vanes and further comprising an inlet in fluid connection with the inlet of the carbamate decomposer and an outlet in fluid connection with the decomposition section, wherein the diameter of the decomposition section is larger than the diameter of the inlet for the Urea-water-carbamate mixture, wherein the diameter of the conical flow distributor is smaller at its inlet than at its outlet.

## Description

### TECHNICAL FIELD OF THE APPLICATION

The present invention is in the field of urea manufacture, in particular in the field of flow distributors for carbamate decomposers in urea plants. It is also in the field of flow distributors per se.

### BACKGROUND OF THE APPLICATION

Carbamate decomposers typically comprise a heat exchanger comprising a plurality of tubes. In some technologies, Urea-water-carbamate is fed at the bottom of the tubes. In the tubes, the carbamate decomposes to form gaseous ammonia and carbon dioxide. The resulting mixture is vented at the top of the decomposers.

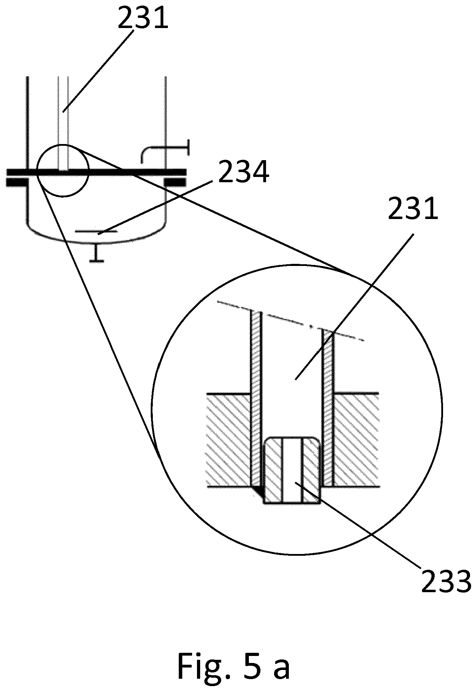

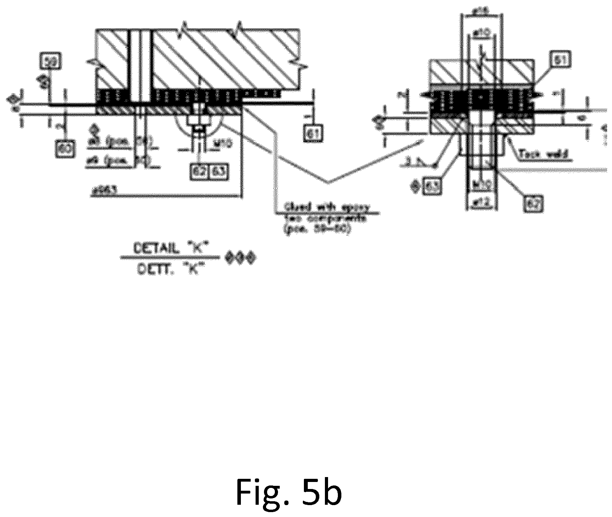

Homogeneous distribution of the Urea-water-carbamate mixture over the tubes in the heat exchanger is critical in order to ensure efficient operation of the decomposer. Accordingly, existing decomposers comprise tubes having a reduced diameter at their lower end. A close-up of such a carbamate decomposer according to the prior art is shown in FIG. 5. As can be seen in this figure, the carbamate decomposer according to the prior art comprises a decomposition section comprising a plurality of tubes (231). The tubes comprise a narrowed lower end (233). The narrowed lower end (233) is effective at ensuring a homogeneous distribution of carbamate-ammonia across the tubes (231) in the carbamate decomposer. However, the narrowed lower ends (233) are prone to clogging, such as iron scale particles created upstream from the decomposer. These particles are created by passive corrosion which occurs during urea synthesis. The particles may obstruct some of the narrow lower end of the tubes of the heat exchanger, which reduces the decomposer's performance; a good, homogeneous distribution across the heat exchanger's tubes is essential for ensuring high performance. The problem can be solved by performing regular preventive maintenance including decomposer shut down, but eliminating the problem altogether would be preferred. Also, the carbamate decomposer according to fig 5 comprises an impingement plate (234) installed to avoid that the flow will feed more the inner tubes and less the outer tubes, thereby creating an uneven distribution of flow through the decomposer, rendering the decomposer less efficient. As a side effect, turbulence is created by the impingement plate (234) which would also result in an uneven distribution of the fluid which requires a compensation using the narrowed lower end (233) of the tubes.

Accordingly, there is a need for carbamate decomposers which are not prone to clogging, which have a low pressure drop, and which do not need regular shutdown.

### SUMMARY OF THE APPLICATION

It is an object of the present invention to provide carbamate decomposers and related methods which meet one or more of the above needs.

Accordingly, provided herein are carbamate decomposers, comprising an inlet for a Urea-water-carbamate mixture, an outlet for a two-phase flow comprising liquid urea, water, un-decomposed carbamate, gaseous ammonia, and gaseous carbon dioxide, a decomposition section, and a fluid distribution section, wherein the fluid distribution section comprises a conical flow distributor, the conical flow distributor comprising a plurality of vanes and further comprising an inlet in fluid connection with the inlet of the carbamate decomposer and an outlet in fluid connection with the decomposition section, wherein the diameter of the decomposition section is larger than the diameter of the inlet for the urea-water-carbamate mixture, wherein the diameter of the conical flow distributor is smaller at its inlet than at its outlet.

In some embodiments, the carbamate decomposer as disclosed herein provides that the conical flow distributor comprises 2 or more vanes.

In some embodiments, the carbamate decomposer as disclosed herein provides that the vanes are arranged concentrically.

In some embodiments, the carbamate decomposer as disclosed herein provides that each vane has a truncated cone shape.

In some embodiments, the carbamate decomposer as disclosed herein provides that the plurality of vanes is arranged to form a plurality of conical fluid ducts between the vanes.

In some embodiments, the carbamate decomposer as disclosed herein provides that the conical fluid ducts are devoid of compartments.

In some embodiments, the carbamate decomposer as disclosed herein provides that the outlet for the two-phase flow is positioned above the inlet for the Urea-water-carbamate mixture.

In some embodiments, the carbamate decomposer as disclosed herein provides that the decomposition section comprises a shell and a plurality of tubes, the tubes being in fluid connection with the fluid distribution section and with the outlet for the two-phase flow.

Also provided herein is a conical flow-distributor, comprising a plurality of vanes, an inlet, and an outlet, wherein the diameter of the conical flow distributor is smaller at the inlet than at the outlet, wherein each vane has a truncated cone shape, and wherein the plurality of vanes is arranged to form a plurality of conical fluid ducts between the vanes, the conical fluid ducts being devoid of compartments.

Also provided herein is a method for converting a Urea-water-carbamate mixture into a two-phase flow comprising liquid urea, water and un-decomposed carbamate, gaseous ammonia, and gaseous carbon dioxide by means of a carbamate decomposer, the method comprising the steps:
- providing a Urea-water-carbamate mixture to an inlet of the carbamate decomposer;
- passing the Urea-water-carbamate mixture through a conical flow distributor, thereby uniformly providing the Urea-water-carbamate mixture to a decomposition section; and,
- decomposing carbamate comprised in the Urea-water-carbamate mixture in the decomposition section, thereby forming a two-phase flow comprising the gaseous ammonia, gaseous carbon dioxide, and liquid urea, water and un-decomposed carbamate.

Also provided herein is the use of a conical flow distributor for uniformly providing a Urea-water-carbamate mixture to a decomposition section in a carbamate decomposer. In particular, the use as disclosed herein provides that the conical flow distributor is a conical flow distributor as disclosed herein.

### DESCRIPTION OF THE FIGURES

The following description of the figures of specific embodiments of the invention is only given by way of example and is not intended to limit the present explanation, its application or use. In the drawings, identical reference numerals refer to the same or similar parts and features.
**Fig. 1** shows a carbamate decomposer (200).
**Fig. 2** shows a close-up of a fluid distribution section (240).
**Fig. 3** is a perspective drawing of a conical flow distributor (100).
**Fig. 4** is a top view of a conical flow distributor (100).
**Fig. 5** is a detail drawing of a carbamate decomposer according to the prior art showing tubes having a narrowed lower end by installing perforated plugs on each tube (5a) or by installing a perforated tray just in front of the tubesheet (5b).

The following reference numerals are used in the description and figures: 100 - flow distributor; 110 - vanes; 120 - outlet of the flow distributor; 130 - inlet of the flow distributor; 140 -fluid ducts; 200 - carbamate decomposer; 210 - inlet of carbamate decomposer; 220 - outlet of carbamate decomposer; 230 - decomposition section; 231 - tubes; 232 - shell-side space; 233 - narrow lower end of tubes; 234 - impingement plate; 240 - fluid distribution section; 250 - decomposer shell; 300 - flow indicators.

### DETAILED DESCRIPTION OF THE APPLICATION

As used below in this text, the singular forms "a", "an", "the" include both the singular and the plural, unless the context clearly indicates otherwise. The terms "comprise", "comprises" as used below are synonymous with "including", "include" or "contain", "contains" and are inclusive or open and do not exclude additional unmentioned parts, elements or method steps. Where this description refers to a product or process which "comprises" specific features, parts or steps, this refers to the possibility that other features, parts or steps may also be present, but may also refer to embodiments which only contain the listed features, parts or steps. The enumeration of numeric values by means of ranges of figures comprises all values and fractions in these ranges, as well as the cited end points. The term "approximately" as used when referring to a measurable value, such as a parameter, an amount, a time period, and the like, is intended to include variations of +/- 10% or less, preferably +/-5% or less, more preferably +/-1% or less, and still more preferably +/-0.1% or less, of and from the specified value, in so far as the variations apply to the invention disclosed herein. It should be understood that the value to which the term "approximately" refers per se has also been disclosed. All references cited in this description are hereby deemed to be incorporated in their entirety by way of reference. Unless defined otherwise, all terms disclosed in the invention, including technical and scientific terms, have the meaning which a person skilled in the art usually gives them. For further guidance, definitions are included to further explain terms which are used in the description of the invention.

The present disclosure relates to carbamate decomposers. The term "carbamate" as used herein is short-hand for ammonium carbamate. The carbamate decomposers provided herein comprise a flow distributor which is positioned in a fluid distribution section, and which serves to uniformly provide a Urea-water-carbamate mixture to a decomposition section. This increases carbamate decomposition efficiency, does not create turbulence, and has a low pressure drop. In addition, the need for narrowing the lower ends of the heat exchanger tubes is obviated such that the system is not prone to clogging.

It should be clear that while the present disclosure in particular refers to flow distributors for carbamate decomposers, same flow distributors as described herein can also be applied for other industrial applications having a similar design as carbamate decomposers such as for instance urea evaporators where a urea solution (comprising urea, water, and minor amounts of NH₃ and CO₂) is fed into the evaporator to concentrate urea solution by evaporating water.

In an embodiment, provided herein is a carbamate decomposer that comprises an inlet for a Urea-water-carbamate mixture, an outlet for a two-phase flow, a decomposition section, and a fluid distribution section. During normal operation, the outlet for the two-phase flow may be positioned above the inlet for the Urea-water-carbamate mixture.

During normal operation, the Urea-water-carbamate mixture is decomposed to form the two-phase flow in the decomposition section. The decomposition section is a heat exchanger. Optionally, the decomposition section comprises a shell and a plurality of tubes, the tubes being in fluid connection with the fluid distribution section and with the outlet for the two-phase flow. The tubes may have a substantially constant cross section along their length. In other words, the tubes are more in particular devoid of narrowed lower ends. The shell-side space may be configured to allow the passage of steam. During normal operation, heat from the steam is used for heating the tubes and their content.

The fluid distribution section comprises a flow distributor. The flow distributor comprises a plurality of vanes, an inlet, and an outlet. In an embodiment, the flow distributor is shaped as a frustum. More in particular, the flow distributor is a conical flow distributor, i.e. a flow distributor shaped as a truncated cone.

The inlet of the flow distributor is in fluid connection with the decomposer's inlet for the Urea-water-carbamate mixture. The outlet of the flow distributor is in fluid connection with the decomposition section. The diameter of the decomposition section is larger than the diameter of the inlet for the Urea-water-carbamate mixture. Also, the diameter of the flow distributor is smaller at its inlet than at its outlet.

Each vane is shaped as a truncated cone. It should be understood that the base and the top of the vanes are open, which allows the passage of fluid.

Also, the vanes can be arranged to form a plurality of frustum-shaped fluid ducts, more in particular conical fluid ducts, between the vanes. In an embodiment, the fluid ducts are devoid of compartments. Differently phrased, in an embodiment, the fluid ducts are devoid of radial partitions. In other words still, the fluid ducts may be continuous. Yet differently put, the fluid ducts may be free of obstructions to fluid flow in the radial direction.

The vanes are positioned into a fixed position and are held there using means available to the skilled person. For instance, the vanes can be assembled together by rods (for example 4 diametrical rods welded together, 2 welded at the proximal end of the vanes and 2 welded at the distal end of the vanes). The set of vanes may be will be inserted into the flow distributor and attached to their using means commonly known to the skilled person, for instance using welded supports.

Additionally or alternatively, the fluid-ducts can be described as shell-shaped ducts having an inner surface and an outer surface. Both the inner surface and the outer surface are shaped as the side-wall of a frustum. The inner surface and the outer surface are the same type of frustum. The inner surface has a smaller base and top as the outer surface. The inner and outer surfaces correspond to the vanes which form the fluid ducts. The base and top of the shell-shaped ducts is open, thus allowing the passage of fluid.

In an embodiment, the ratio of the areas of the transversal cross sections of any two conical fluid ducts is kept constant along the lateral direction of the conical fluid distributor (100). This allows maintaining a uniform fluid velocity profile across the transversal cross section of the conical fluid distributor. Accordingly, the flow distributor forms a smooth transition between the small-diameter inlet and the large-diameter decomposition section. This enhances the homogeneity of the Urea-water-carbamate mixture flow into the decomposition section without creating a significant pressure drop. This in turn increases the decomposition efficiency of the decomposer without substantially increasing the energy consumption. In addition, the system has very little risk of clogging.

In some embodiments, the flow distributor comprises 2 or more vanes.

In some embodiments, the vanes are arranged concentrically.

Further provided herein is a flow distributor comprising a plurality of vanes, an inlet, and an outlet. The diameter of the conical flow distributor is smaller at the inlet than at the outlet.

In an embodiment, each vane is shaped as a frustum. More in particular, each vane is shaped as a truncated cone.. It should be understood that the base and the top of the vanes are open, which allows the passage of fluid. Further details of the vanes have been described above. The shape of the vanes may be selected to match the shape of the fluid channel in which the flow distributor is positioned. This allows efficient distribution of fluid flow in the fluid channel.

In an embodiment, the vanes are arranged to form a plurality of frustum-shaped fluid ducts, more in particular conical fluid ducts, between the vanes. More in particular, the fluid ducts are devoid of compartments. Further details of the fluid ducts have been described above.

Further provided herein is the use of a flow distributor for uniformly providing a Urea-water-carbamate mixture to a decomposition section in a carbamate decomposer. More in particualr, the flow distributor is a flow distributor as described above.

Further provided is a method for converting a Urea-water-carbamate mixture into a two-phase flow by means of a carbamate decomposer as described herein. The two-phase flow comprises liquid urea, water, un-decomposed carbamate, gaseous ammonia, and gaseous carbon dioxide. The method involves providing a Urea-water-carbamate mixture to the inlet of the carbamate decomposer. The Urea-water-carbamate mixture is passed through a flow distributor. The flow distributor may be a conical flow distributor. However, other frustum-shaped flow distributors may be used as well. Such flow distributors have been described above. The flow distributor ensures that the Urea-water-carbamate mixture is uniformly provided to the decomposition section.

In the decomposition section, the Urea-water-carbamate mixture is decomposed. As a result, a two-phase flow comprising gaseous ammonia, gaseous carbon dioxide, and liquid urea, water and un-decomposed carbamate is formed. The decomposition in the decomposition section is facilitated by providing heat to the Urea-water-carbamate mixture. In particular, the Urea-water-carbamate mixture and the two-phase flow upward through a plurality of tubes in the decomposition section. Optionally, the heat is provided by means of steam which flows through a shell-side space in the decomposition section. The shell-side space is the space between the tubes and the shell of the decomposition section.

After the two-phase flow leaves the decomposition section, the two-phase flow can be vented through the outlet of the carbamate decomposer. During normal operation, the vented two-phase flow is subsequently treated in a device for separating the liquid urea, water and un-decomposed carbamate from the gaseous ammonia and carbon dioxide. An example of a suitable device for separating the two-phase flow is a cyclone.

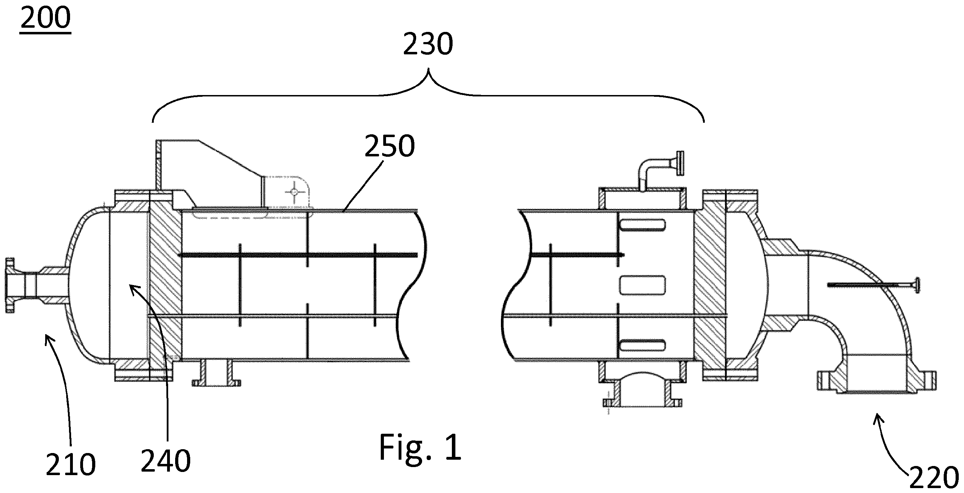

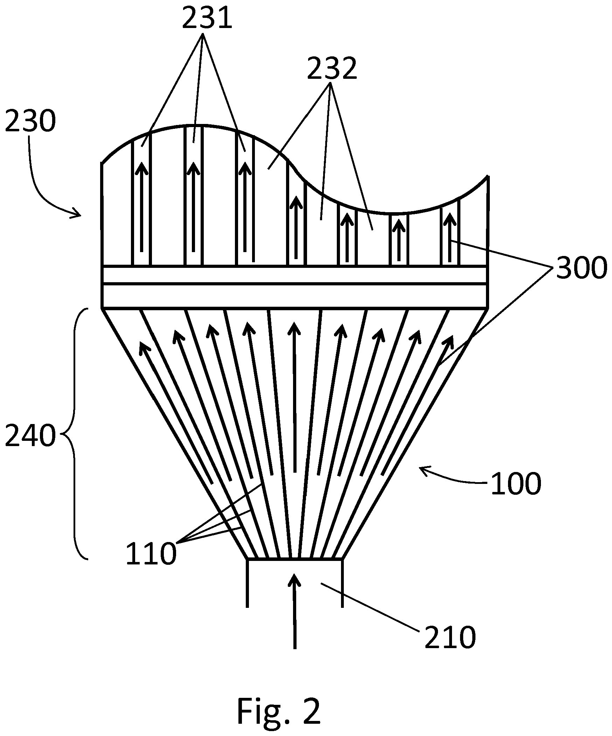

An exemplary embodiment of a carbamate decomposer (200) according to the present application is shown in Figs. 1 and 2. This carbamate decomposer (200) comprises a decomposition section (230) in which carbamate comprised in a Urea-water-carbamate mixture is decomposed to form ammonia and carbon dioxide. The Urea-water-carbamate mixture is fed to the bottom of the decomposition section (230) via an inlet (210). The decomposition section (230) consists of a heat exchanger comprising a plurality of tubes (231) and a shell (250). Between the tubes (231) and the shell (250), a shell-side space (232) is formed. In the decomposition section (230), the Urea-water-carbamate mixture flows upward through the tubes (231), and it is heated by means of steam which flows through the shell-side space (232). Carbamate decomposes in the decomposition section (230) to form ammonia and carbon dioxide. Urea does not react substantially in the decomposition section (230). Accordingly, a two-phase mixture consisting of ammonia, carbon dioxide, and urea is formed in the decomposition section (230). This two-phase mixture flows upward until it reaches the outlet (220) of the carbamate decomposer (200). At the outlet (220), the two-phase mixture exits the carbamate decomposer (200).

Homogeneous flow through the tubes (231) in the carbamate decomposer (200) is crucial in order to ensure operational efficiency. To ensure homogeneous flow through the tubes (231), a flow distributor (not shown in Fig. 1) is provided in a fluid distribution section (240). The flow distributor ensures homogeneous distribution of a Urea-water-carbamate mixture to the tubes (231).

Fig. 2 shows a close-up of a carbamate decomposer. In particular, Fig. 2 shows an inlet (210), a fluid distribution section (240), and a decomposition section (230). The conical flow distributor (100) is installed in the fluid distribution section (240). The carbamate-urea mixture flows through the inlet (210) into the fluid distribution section, where it is guided by the truncated cone-like vanes (110) of the conical flow distributor (100). The vanes (110) extend longitudinally in the direction of fluid flow between the inlet and the distribution section (240). The vanes (110) guide the fluid flow and provide a smooth transition between the inlet (210) which has a small diameter of e.g. 30.0 cm, and the decomposition section (230) which has a larger diameter of e.g. 100.0 cm. Accordingly, the carbamate-urea mixture is brought to the tubes (231) in the decomposition section (230) in a uniform way and with a minimal pressure drop. The flow of the carbamate-urea mixture is indicated by means of flow indicators (300). In the decomposition section (230), the Urea-water-carbamate mixture is decomposed under influence of heat provided by means of steam flowing through the shell-side space (232).

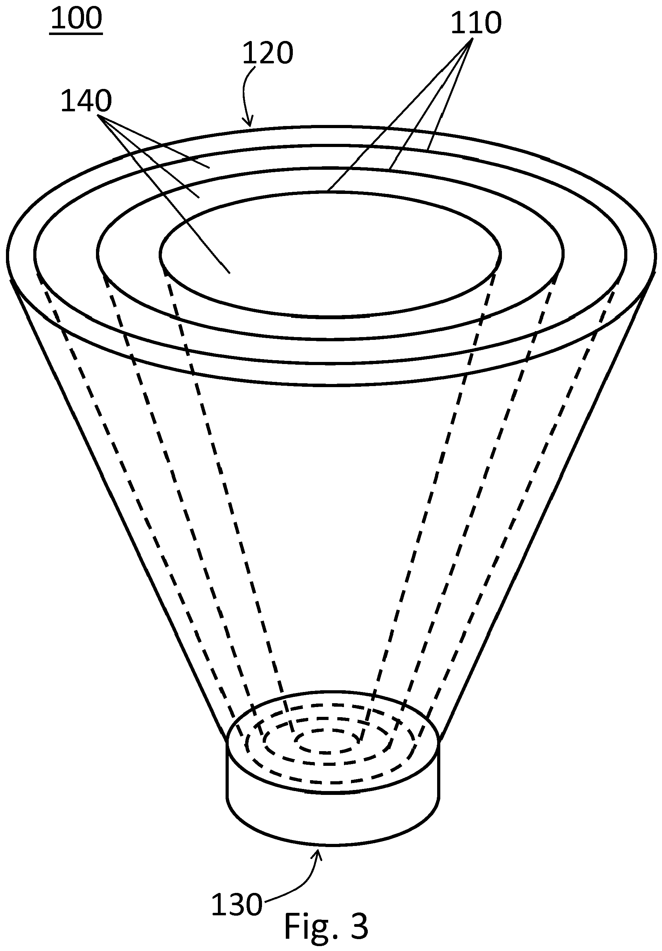

In Fig. 3, a perspective view of the conical flow distributor (100) is shown. It comprises an inlet (130) and an outlet (120). The conical flow distributor (100) is shaped as a truncated cone. The outlet (120) corresponds to the base of the truncated cone and the inlet (130) corresponds to the truncated apex of the truncated cone. The conical flow distributor (100) comprises three concentric vanes (110) which define conical fluid ducts (140) through which a Urea-water-carbamate mixture can flow.

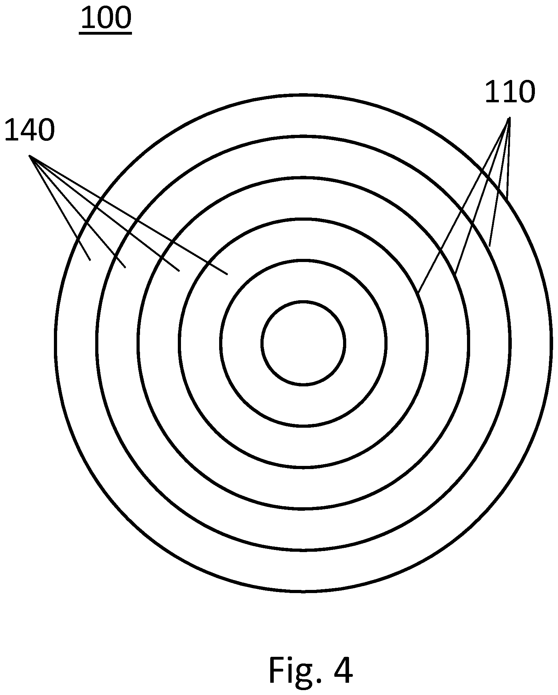

Fig. 4 shows a transversal cross section of a conical flow distributor (100). The vanes (110) are shown as concentric circles. Between the vanes (110), conical fluid ducts (140) are formed. Along the length of the conical flow distributor (100), the radius of the vanes (110) gradually increases from the inlet (130) of the conical flow distributor (100) to the outlet (120) of the conical flow distributor (100). Along the lateral direction of the conical fluid distributor (100) the fluid velocity gradually decreases during normal use when going from the inlet (130) to the outlet (120) of the conical fluid distributor (100). The rate of increase of the radii of the vanes (110) is chosen to maintain a constant fluid velocity across the transversal cross section of the conical fluid distributor (100) during normal use.
In order to maintain a constant fluid velocity across the transversal cross section of the conical fluid distributor (100), the ratio of the areas of the transversal cross sections of any two conical fluid ducts is kept constant along the lateral direction of the conical fluid distributor (100).

## Claims

1. A carbamate decomposer (200), comprising an inlet (210) for a Urea-water-carbamate mixture, an outlet (220) for a two-phase flow comprising liquid urea, water, un-decomposed carbamate, gaseous ammonia, and gaseous carbon dioxide, a decomposition section (230), and a fluid distribution section (240), wherein the fluid distribution section (240) comprises a conical flow distributor (100), the conical flow distributor (100) comprising a plurality of vanes (110) and further comprising an inlet (130) in fluid connection with the inlet (210) of the carbamate decomposer and an outlet (120) in fluid connection with the decomposition section (230), wherein the diameter of the decomposition section (230) is larger than the diameter of the inlet (210) for the urea-carbamate mixture, wherein the diameter of the conical flow distributor (100) is smaller at its inlet (130) than at its outlet (120).

2. The carbamate decomposer (200) according to claim 1, wherein the conical flow distributor (100) comprises between 2 and 4 vanes (110).

3. The carbamate decomposer (200) according to claim 1 or 2, wherein the vanes (110) are arranged concentrically.

4. The carbamate decomposer (200) according to any one of claims 1 to 3, wherein each vane has a truncated cone shape.

5. The carbamate decomposer (200) according to any one of claims 1 to 4, wherein the plurality of vanes (110) is arranged to form a plurality of conical fluid ducts (140) between the vanes (110).

6. The carbamate decomposer according to claim 5, wherein the conical fluid ducts (140) are devoid of compartments.

7. The carbamate decomposer (200) according to any one of claims 1 to 6, wherein the outlet (220) for the two-phase flow is positioned above the inlet (210) for the Urea-water-carbamate mixture.

8. The carbamate decomposer (200) according to any one of claims 1 to 7, wherein the decomposition section (230) comprises a shell (250) and a plurality of tubes (231), the tubes being in fluid connection with the fluid distribution section (240) and with the outlet (220) for the two-phase flow.

9. A conical flow-distributor (100), comprising a plurality of vanes (110), an inlet (130), and an outlet (120), wherein the diameter of the conical flow distributor (100) is smaller at the inlet (130) than at the outlet (120), wherein each vane has a truncated cone shape, and wherein the plurality of vanes (110) is arranged to form a plurality of conical fluid ducts (140) between the vanes (110), the conical fluid ducts (140) being devoid of compartments.

10. A method for converting a Urea-water-carbamate mixture into a two-phase flow comprising liquid urea, water, un-decomposed carbamate, gaseous ammonia, and gaseous carbon dioxide by means of a carbamate decomposer (200), the method comprising the steps:
- providing a Urea-water-carbamate mixture to an inlet (210) of the carbamate decomposer (200);
- passing the Urea-water-carbamate mixture through a conical flow distributor (100), thereby uniformly providing the Urea-water-carbamate mixture to a decomposition section (230); and,
- decomposing carbamate comprised in the Urea-water-carbamate mixture in the decomposition section (230), thereby forming a two-phase flow comprising the gaseous ammonia, gaseous carbon dioxide, and liquid urea, water and un-decomposed carbamate.

11. Use of a conical flow distributor for uniformly providing a Urea-water-carbamate mixture to a decomposition section (230) in a carbamate decomposer (200).

12. Use according to claim 11, wherein conical flow distributor is a conical flow distributor according to claim 9.
